# FASCICULE DE BREVET EUROPEEN

(11) **EP 0 526 512 B1**
(45) Date de publication et mention de la délivrance du brevet: **09.11.1994**
(21) Numéro de dépôt: 91908159.6
(22) Date de dépôt: 10.04.1991
(51) Int. Cl.: A61F 9/00, A61J 1/00

(54) **PROCEDE ET CONDITIONNEMENT POUR CONSERVER ET DISTRIBUER PAR PORTIONS DU LIQUIDE STERILE**
VERFAHREN SOWIE VERPACKUNG ZUM AUFBEWAHREN UND ABGEBEN VON STERILEN FLÜSSIGKEITSMENGEN
METHOD AND PACKAGING FOR PRESERVING AND DISPENSING PORTIONS OF A STERILE LIQUID

(30) Priorité: 27.04.1990 FR 9005369
(43) Date de publication de la demande: 10.02.1993
(73) Titulaire: TRANSPHYTO S.A., 63000 Clermont Ferrand (FR)
(72) Inventeur: LONTRADE, Jean-Pierre, F-63000 Clermont-Ferrand (FR); CHIBRET, Henri, F-63000 Clermont-Ferrand (FR)
(74) Mandataire: Thibon-Littaye, Annick
(86) Numéro de dépôt international: FR9100291
(87) Numéro de publication internationale: WO9116868

(56) Documents cités:
- EP-A- 401 022
- WO-A-90/05110
- DE-A- 3 628 197
- FR-A- 2 422 569

## Description

La présente invention concerne l'industrie du conditionnement et plus particulièrement celui des substances stériles à distribuer par fractions, telles que les collyres.

On a conditionné de telles substances dans des récipients rigides avec compte-gouttes séparés, puis dans des récipients élastiquement déformables avec ajutage stilligoutte, avec toujours le risque de contamination du liquide inutilisé, conservé pour une utilisation ultérieure dans le récipient après contact avec l'atmosphère. On a ensuite proposé d'utiliser des récipients dont la stérilité du contenu est préservée par un filtre stérilisant interposé dans l'ouverture du récipient, pouvant être constitué par une céramique, mais le plus souvent par une membrane microporeuse en matière plastique comme décrit notamment dans le brevet français n° 2 422 569. Il reste cependant inévitablement un peu de liquide à l'extérieur de la membrane après une distribution et ce liquide n'échappe pas ainsi à une contamination extérieure jusqu'à la distribution ultérieure du liquide conservé stérile qui est alors contaminé par ce liquide resté à l'extérieur.

Les filtres stérilisants constitués par des membranes microporeuses ont la propriété de devenir imperméables à l'air à l'état mouillé sauf sous forte pression. Cette propriété a été considérée jusqu'ici comme un inconvénient et le brevet français 2 422 569 préconise de ménager dans la membrane une zone imperméable au liquide mais perméable à l'air, par exemple par hydrofugation locale au silicone dans le cas de liquide aqueux, ce qui permet à un récipient à déformation élastique d'aspirer de l'air pour compenser le volume de liquide distribué et de reprendre ainsi sa forme initiale. Mais la pression différentielle de part et d'autre de la membrane permettant l'aspiration d'air est inférieure à celle qui permettrait l'aspiration du liquide et, de ce fait, le liquide qui a traversé la membrane ne peut être récupéré et conservé stérile.

A défaut d'utiliser une membrane à zones perméables à l'air ou au liquide, ce brevet français 2 422 569 signale la possibilité d'utiliser des récipients déformables non élastiques, par exemple en aluminium mince, dont le volume diminue à mesure des distributions, mais de tels récipients sont incapables d'aspirer le liquide qui a traversé la membrane et reste à son contact après une distribution.

L'invention tire au contraire avantage de ce prétendu inconvénient pour éviter toute perte et contamination du liquide restant au contact d'une membrane microporeuse après une distribution.

Le but est atteint en maintenant la membrane humectée par le liquide entre les distributions successives, et donc imperméable à l'air, tout en la soumettant en permanence à une pression différentielle d'aspiration du liquide de valeur insuffisante pour permettre une aspiration à force de l'air à travers la membrane mouillée.

La pression différentielle minimum permettant à l'air de traverser une membrane microporeuse mouillée est déterminée couramment par le test dit "du point de bulle" par lequel on soumet une face de la membrane mouillée à une pression d'air croissante et où l'on retient comme valeur spécifique du "point de bulle" la pression différentielle qui provoque l'apparition d'un flux de bulles continu en aval de la membrane. Les valeurs des points de bulle spécifiques à chaque type de membrane sont généralement fournis par leurs fabricants.

L'invention a ainsi pour objet un procédé permettant de distribuer par portions du liquide stérile enfermé dans un récipient sous contrôle d'une membrane microfiltrante perméable au liquide mais imperméable à l'air à l'état mouillé, interposée dans le goulot, sans contamination du liquide restant entre distributions ladite membrane etant humectée avec le liquide de l'intérieur du récipient avant la première distribution, et la quantité de liquide désirée étant distribuée à travers la membrane en exerçant une pression sur le liquide dans le récipient. Ce procédé est caractérisé en ce qu'on aspire sélectivement dans le récipient après distribution et au travers de la membrane le liquide restant extérieurement au contact de la membrane, sans aspiration d'air et en ce qu'on conserve la membrane humectée de liquide entre les distributions successives.

Suivant un tel procédé, on peut entreposer le liquide dans un récipient élastiquement déformable à la main, dont la récupération de forme après déformation crée entre les faces de la membrane une pression différentielle de valeur inférieure à celle du point de bulle de la membrane mais au moins égale à celle nécessaire à l'aspiration du liquide à travers la membrane.

En variante, on peut enfermer dans le récipient, avec le liquide, un volume gazeux inerte vis-à-vis du liquide dont la compression par la déformation manuelle du récipient permet la distribution de pratiquement la totalité du liquide à travers la membrane.

En effet, les récipients classiques ne sont pas élastiquement déformables en totalité, notamment au goulot et il n'est alors pas possible d'expulser la totalité du liquide par simple pression sur la partie déformable, sauf à transmettre au liquide la pression exercée sur la paroi déformable par l'intermédiaire d'une quantité suffisante d'air ou autre gaz enfermé' dans le récipient conjointement au liquide.

L'invention a également pour objet un conditionnementdistributeur de liquide stérile, mettant en oeuvre le procédé de l'invention, notamment de liquide sensible au contact de l'air, comprenant un récipient à paroi élastiquement déformable par compression manuelle, dont l'ouverture distributrice est contrôlée par une membrane microfiltrante, perméable au liquide mais imperméable à l'air à l'état mouillé, conditionnement caractérisé en ce que la force de récupération élastique de forme de la paroi du récipient après déformation manuelle crée entre les faces de la membrane une pression différentielle inférieure à la pression différentielle du point de bulle permettant à l'air de traverser la membrane mouillée, grâce à quoi, après expulsion de liquide à l'extérieur à travers la membrane sous l'effet d'une pression exercée extérieurement sur le récipient, le liquide restant au contact de la face extérieure de la membrane lorsque cesse la pression extérieure, retourne sélectivement dans le récipient à travers la membrane par aspiration sous l'effet de la dépression créée dans le récipient par la reprise de forme de sa paroi, sans que de l'air extérieur puisse être aspiré concomitamment ; et en ce qu'il comporte des moyens propres à imposer un stockage en position retournée de sorte à maintenir la membrane humectée de liquide entre des distributions successives.

Dans un tel conditionnement, généralement la pression différentielle du point de bulle de la membrane est d'environ 4 bars, tandis que la récupération élastique de forme du récipient après déformation est d'environ 0,003 bar.

Suivant un mode de réalisation avantageux, la membrane est coiffée extérieurement par un embout distributeur maintenant la face externe de la membrane contre toute déformation préjudiciable à son intégrité, tout en permettant l'évacuation du liquide ayant traversé la membrane vers un ajutage de distribution capillaire, le volume total maximum de liquide entre la face externe de la membrane et l'extrémité de l'ajutage étant d'environ 1 à 3 gouttes.

Un conditionnement suivant l'invention peut avantageusement comprendre un capuchon, obturateur de l'embout de distribution du liquide, qui constitue un socle pour le stockage du récipient en position retournée maintenant ainsi le liquide du récipient en contact permanent avec la membrane.

Selon un mode de réalisation préféré du conditionnement selon l'invention, celui-ci comporte avantageusement des moyens propres à en imposer le stockage en position retournée de sorte à ce que la membrane soit maintenue à l'état mouillé même pendant des périodes de stockage de longue durée en l'absence de capuchon obturateur de l'embout.

De tels moyens confèrent avantageusement au conditionnement une instabilité dans une position où l'embout distributeur est dirigé vers le haut et sont de préférence associés à des moyens d'appui propres à conférer au conditionnement une stabilité en position retournée.

Le fait de prévoir de tels moyens permet notamment d'éluder un problème d'assèchement de la membrane par évaporation notamment si l'utilisateur oublie de refermer l'embout par le capuchon obturateur pendant une période de stockage longue. Cela peut également permettre dans d'autres formes de réalisation de ne pas prévoir de capuchon obturateur.

Selon une caractéristique particulièrement avantageuse du conditionnement selon l'invention, ledit récipient à paroi élastiquement déformable comporte une surface postérieure constituant une surface d'appui instable contribuant à imposer un stockage en position retournée.

Le conditionnement selon l'invention comporte avantageusement une gaine rigide de protection dudit récipient à paroi élastiquement déformable, ladite gaine étant de préférence ouverte à son extrémité postérieure de sorte que ledit récipient dépasse de la gaine en position de repos permettant ainsi de conserver l'instabilité du conditionnement en position autre que retournée.

Selon une variante de réalisation du conditionnement selon l'invention, la gaine rigide est découpée depuis son extrémité postérieure de manière à ne pas présenter une surface d'appui pour le stockage du conditionnement, par exemple en étant largement découpée en biseau. Dans ce cas, la gaine elle-même constitue les moyens propres à imposer le stockage en position retournée.

En variante, le conditionnement peut comporter un tube plongeant jusqu'au fond du récipient et prolongé extérieurement par un col de cygne terminé par un embout distributeur renfermant la membrane.

Suivant une autre variante, le conditionnement comporte une pompe manuelle sans clapet mécanique, incorporée à un récipient rétrécissable qui refoule le liquide du récipient à travers une membrane perméable seulement au liquide, formant valve anti-retour sélective contre l'action d'une force élastique de rappel tarée à une force inférieure à celle du point de bulle de la membrane.

L'invention pourra être mieux comprise par l'examen et la description détaillée des dessins annexés qui représentent plusieurs modes de réalisation, choisis simplement à titre d'exemples parmi les nombreuses formes d'exécution, adaptation, et variantes de l'invention accessible à un technicien averti.

Sur ces dessins :
- la figure 1 est une vue schématique, en élévation et en coupe axiale d'un premier mode de réalisation de conditionnement suivant l'invention ;
- la figure 2 est une vue analogue à celle de la figure 1 d'un deuxième mode de réalisation ;
- la figure 3 est une variante de réalisation du conditionnement représentée à la figure 2 ;
- la figure 4 est une vue analogue aux précédentes d'un quatrième mode de réalisation ;
- la figure 5 est une vue schématique, par dessus, d'un conditionnement identique à celui de la figure 4, mais avec un tube distributeur décalé de 90°.

Sur ces figures, les éléments correspondants sont désignés par les mêmes références numériques, éventuellement affectées d'un indice. Les dimensions et les proportions respectives de ces éléments peuvent ne pas être respectées pour une meilleure lisibilité des dessins.

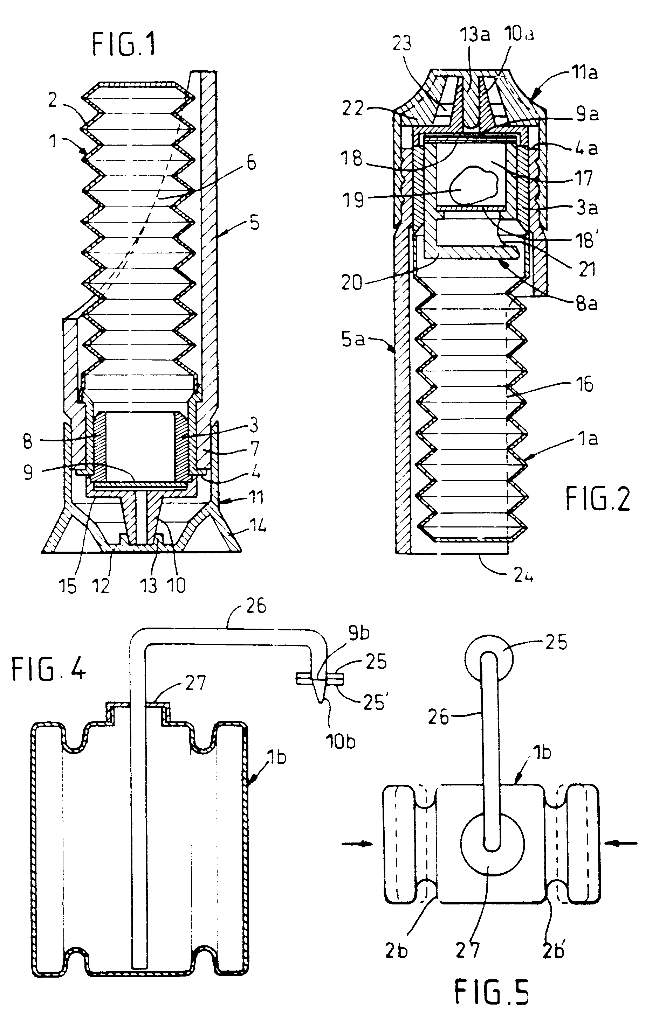

Le conditionnement-distributeur de liquide stérile représenté sur la figure 1 comprend principalement un récipient 1 en matière plastique soufflée de forme cylindrique, dont la partie postérieure 2 est plissée transversalement à l'axe, comme un accordéon. La partie antérieure 3 est cylindrique de révolution et se termine pas une collerette 4 entourant le goulot.

Le récipient 1 est partiellement entouré par une gaine ouverte 5 dont la partie postérieure 6 est largement taillée en biseau, laissant ainsi librement accès à la partie plissée 2 du récipient. La partie antérieure 7 de la gaine est cylindrique de révolution et reçoit à frottement ferme la partie antérieure 3 du récipient dont la collerette 4 est assujettie par collage, soudure thermique ou par ultrason ou par friction à la tranche du bord supérieur de la gaine 5.

A l'intérieur de la partie antérieure 3 du récipient est inséré à frottement étanche un bouchon tubulaire ouvert 8 dont l'ouverture extérieure est fermée par une membrane microfiltrante 9 scellée par collage, soudure thermique ou par ultrasons de sa périphérie sur la tranche de l'ouverture du bouchon 8.

Un embout stilligoutte 10 est encliqueté de façon étanche sur le bord latéral de l'ouverture du bouchon 8 et la périphérie de sa base vient porter sur la collerette 4 du récipient 1, ménageant un faible intervalle entre son fond plat 15 et la membrane 9 pour l'écoulement du liquide.

Un capuchon 11 tubulaire borgne amovible est inséré à frottement doux sur la partie supérieure cylindrique 7 de la gaine 5. Son fond 12 présente intérieurement une protubérance annulaire 14 qui vient emboîter de façon étanche l'extrémité ouverte de l'embout stilligoutte 10. Extérieurement, le fond 12 est prolongé et entouré par un épanouissement tronconique 14 qui sert de socle pour poser le conditionnement en position retournée, comme représenté sur la figure 1, sur une table ou autre surface plane, alors que la base biseautée de la gaine 5 empêche de poser le conditionnement verticalement dans l'autre sens, l'appui offert par l'extrémité libre de la gaine sur une telle surface étant trop étroit. Ainsi, la membrane 9 reste mouillée par le liquide du récipient entre les utilisations.

Pour le conditionnement des collyres, par exemple, on utilisera avantageusement une membrane microfiltrante hydrophile en esters de cellulose en forme de disque circulaire d'environ 1 cm de diamètre, dont les pores ont une dimension moyenne de 0,22 µm ± 0,02 et dont le point de bulle est de 3,87 bars, telle celle vendue sous la dénomination commerciale de MF-MILLIPORE type GS par la Société Française dite MILLIPORE S.A. On l'associera, par exemple, à un récipient d'environ 10 cm³ de capacité utile en polyéthylène basse densité soufflé d'environ 15 mm de diamètre extérieur, plissé par une dizaine de soufflets transversaux d'environ 12 mm de diamètre interne et d'une épaisseur moyenne de paroi d'environ 0,5 mm. La dépression créée par la reprise de forme d'un tel récipient après compression axiale des soufflets est d'environ 0,002 à 0,004 bar.

Le volume délimité entre la membrane et l'embouchure du stilligoutte est d'une à trois gouttes et de préférence 1 goutte.

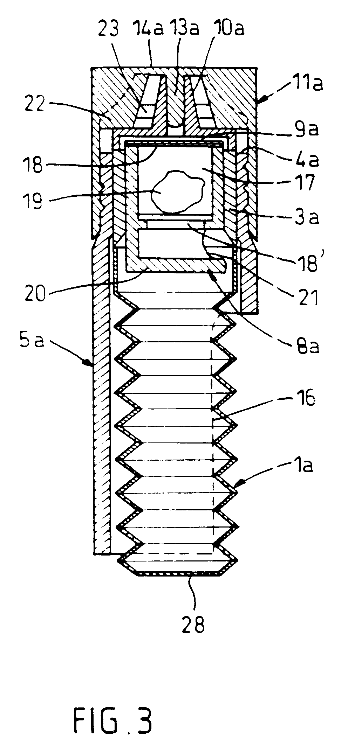

Dans les modes de réalisation présentés sur les figures 2 et 3, on retrouve plusieurs éléments analogues à ceux du premier mode de réalisation de la figure 1 : un récipient plissé accordéon 1a, entouré d'une gaine 5a coiffée d'un capuchon 11a. Mais ici, la gaine cylindrique ne présente qu'une fente latérale longitudinale 16 dont la largeur permet le passage d'un doigt de l'utilisateur. Cette gaine dans le mode de réalisation représenté à la figure 2 laisse à l'extrémité ouverte de la gaine une assise semi-circulaire 24 suffisante pour supporter le conditionnement debout sur une table ou autre surface plane, comme représenté sur la figure 2.

Dans la variante représentée à la figure 3, la gaine 5a est dimensionnée en longueur, de sorte que l'extrémité postérieure du récipient 1a dépasse de l'extrémité ouverte de la gaine, et l'extrémité postérieure du récipient 1a se termine par une surface postérieure (28) constituant une surface d'appui instable de sorte à imposer un stockage du conditionnement en position retournée.

La membrane 9a est fixée en bout d'un bouchon 8a coulissant de façon étanche dans la partie supérieure cylindrique 3a du récipient 1a. Ce bouchon constitue une nacelle avec un logement interne 17 délimité par deux tamis 18, 18' entre lesquels on peut disposer une substance 19 soluble dans le liquide ou en modifiant ou absorbant certains constituants. La base du bouchon est fermée par une cloison 20 et une ouverture latérale 21 débouche entre le tamis 18' et la cloison 20. Le tamis 18 sert de support à la membrane 9a sur toute sa surface utile et l'embout stilligoutte 10a présente au contact de la membrane des cannelures 22 maintenant la membrane sans nuire à l'écoulement du liquide.

Avant la première utilisation, le capuchon 11a n'est que partiellement vissé et le bouchon 8a n'est que partiellement enfoncé dans le col du récipient 1a afin que la cloison inférieure 20 isole le logement 17 et son contenu du liquide à l'intérieur du récipient. Le capuchon 11a présente un téton axial 13a inséré de façon étanche dans le canal du stilligoutte 10a qui isole la membrane et le logement 17 de l'extérieur. Il comporte en outre des ailettes radiales internes 23 qui prennent appui sur la face extérieure du stilligoutte pour l'enfoncer ainsi que le bouchon 8a lors du vissage dans le col du récipient pour la première utilisation jusqu'à butée sur la collerette 4a du récipient, comme représenté sur les figures 2 et 3, mettant ainsi l'intérieur du bouchon en communication avec l'intérieur du récipient.

Pendant les périodes de stockage du conditionnement, le téton axial 13a est engagé de manière étanche dans le canal ou ajutage du stilligoutte 10a et permet ainsi que la membrane reste mouillée en évitant son assèchement par de l'air venant de l'extérieur.

Un tel dispositif à nacelle mobile formant sas est décrit dans la demande de brevet européen n° 89 40 29 16 4.

Dans le mode de réalisation tel que représenté à la figure 3, le capuchon 11a se termine en une surface d'appui 14a pour le stockage du conditionnement en position retournée.

Dans le quatrième mode de réalisation représenté sur les figures 4 et 5, la membrane 9b est fixée entre deuxflasques 25, 25' assemblées, issues l'une d'un embout stilligoutte terminal 10b, l'autre de l'extrémité d'un tube 26 dont l'autre extrémité plonge axialement au fond d'un récipient 1b de section générale rectangulaire englobant deux soufflets 2b, 2b' faisant symétriquement le tour du récipient parallèlement à son axe. Les tracés en pointillés indiquent la déformation des soufflets lorsqu'on comprime le récipient latéralement suivant les flèches de la figure 4. Le tube 26 traverse de façon étanche un opercule 27 obturant de façon étanche le goulot du récipient, tout en étant éventuellement orientable parallèlement aux soufflets (figure 4) ou transversalement (figure 5) suivant les commodités de l'utilisateur. On peut envisager de remplir totalement de liquide les récipients, mais il paraît plus pratique de ne les remplir que partiellement, par exemple aux 2/3 ou à la moitié pour réserver un espace libre que l'on remplira d'air ou autre gaz, notamment d'azote, stérile, afin de permettre l'évacuation de la quasi totalité du liquide. La membrane mouillée par le liquide empêche la fuite de gaz à l'extérieur.

## Revendications

1. Procédé pour distribuer par portions du liquide stérile enfermé dans un récipient (1,1a,1b) sous contrôle d'une membrane microfiltrante (9,9a,9b) perméable au liquide mais imperméable à l'air à l'état mouillé, interposée dans le goulot, sans contamination du liquide restant entre chaque distribution, ladite membrane (9,9a,9b) étant humectée avec le liquide de l'intérieur du récipient (1,1a,1b) avant la première distribution, et la quantité de liquide désirée étant distribuée à travers la membrane (9,9a,9b) en exerçant une pression sur le liquide dans le récipient (1,1a,1b), caractérisé en ce qu'on aspire sélectivement dans le récipient (1,1a,1b), après distribution et au travers de la membrane (9,9a,9b), le liquide restant extérieurement au contact de cette dernière, sans aspiration d'air, et en ce qu'on conserve la membrane (9,9a,9b) humectée de liquide entre les distributions successives.

2. Procédé selon la revendication 1, caractérisé en ce qu'on entrepose le liquide dans un récipient (1,1a,1b) élastiquement déformable à la main, dont la récupération de forme après déformation crée entre les faces de la membrane une pression différentielle de valeur inférieure à celle du point de bulle de la membrane (9,9a,9b) mais au moins égale à celle nécessaire à l'aspiration du liquide à travers la membrane (9,9a,9b).

3. Procédé selon l'une des revendications 1 et 2, caractérisé en ce qu'on enferme dans le récipient (1,1a,1b) avec le liquide, un volume gazeux inerte vis-à-vis du liquide dont la compression par la déformation manuelle du récipient (1,1a,1b) permet la distribution de pratiquement la totalité du liquide à travers la membrane (9,9a,9b).

4. Conditionnement-distributeur de liquide stérile selon le procédé de l'une des revendications 1 à 3 notamment de liquide sensible au contact de l'air, comprenant un récipient (1, 1a, 1b) à paroi élastiquement déformable par compression manuelle, dont l'ouverture distributrice est contrôlée par une membrane microfiltrante (9,9a,9b), perméable au liquide mais imperméable à l'air à l'état mouillé, conditionnement caractérisé en ce que la force de récupération élastique de forme de la paroi du récipient (1,1a,1b) après déformation manuelle crée entre les faces de la membrane (9,9a,9b) une pression différentielle inférieure à la pression différentielle du point de bulle permettant à l'air de traverser la membrane mouillée, grâce à quoi, après expulsion de liquide à l'extérieur à travers la membrane sous l'effet d'une pression exercée extérieurement sur le récipient, le liquide restant au contact de la face extérieure de la membrane lorsque cesse la pression extérieure, retourne sélectivement dans le récipient à travers la membrane par aspiration sous l'effet de la dépression créée dans le récipient par la reprise de forme de sa paroi, sans que de l'air extérieur puisse être aspiré concomitamment ; et en ce qu'il comporte des moyens propres à imposer un stockage en position retournée de sorte à maintenir la membrane humectée de liquide entre des distributions successives.

5. Conditionnement selon la revendication 4, caractérisé en ce que ledit récipient (1,1a) comporte une surface postérieure (28) constituant une surface d'appui instable.

6. Conditionnement selon la revendication 4 ou 5, caractérisé en ce qu'il comporte une gaine rigide (5,5a) de protection dudit récipient à paroi élastiquement déformable (1,1a), ladite gaine étant découpée de manière à autoriser l'accès audit récipient pour y exercer une pression propre à expulser une portion du liquide.

7. Conditionnement selon les revendications 5 et 6, caractérisé en ce que ladite gaine rigide (5a) est ouverte à son extrémité postérieure, de sorte que ledit récipient (1a) dépasse de la gaine en position de repos.

8. Conditionnement selon d'une des revendications 6 et 7, caractérisé en ce que ladite gaine (5b) est découpée de manière à ne pas présenter une surface d'appui pour le stockage du conditionnement.

9. Conditionnement selon la revendication 8, caractérisé en ce que ladite gaine (5) est largement taillée en biseau depuis son extrémité postérieure.

10. Conditionnement selon l'une quelconque des revendications 4 à 9, caractérisé en ce que la membrane (9,9a) est coiffée extérieurement par un embout distributeur (10,10a) maintenant la face externe de la membrane contre toute déformation préjudiciable à son intégrité, tout en permettant l'évacuation du liquide ayant traversé la membrane vers un ajutage de distribution capillaire, volume total maximum de liquide entre la face externe de la membrane et l'extrémité de l'ajutage étant d'environ 1 à 3 gouttes.

11. Conditionnement selon l'une quelconque des revendcations 4 à 10, caractérisé en ce qu'il comprend un capuchon (11,11a), obturateur de l'embout (10,10a) de distribution du liquide, qui constitue un socle (14,14a) pour le stockage du récipient en position retournée maintenant ainsi le liquide du récipient en contact permanent avec la membrane.

12. Conditionnement selon l'une des revendications 4 ou 10, caractérisé en ce qu'il comporte un tube (26) plongeant jusqu'au fond du récipient et prolongé extérieurement par un col de cygne terminé par un embout distributeur (10b) renfermant la membrane (9b).

13. Conditionnement selon l'une des revendications 4 à 12, caractérisé en ce qu'il comporte une pompe manuelle sans clapet mécanique, incorporée à un récipient rétrécissable qui refoule le liquide du récipient à travers une membrane perméable seulement au liquide formant valve antiretour sélective contre l'action d'une force élastique de rappel tarée à une force inférieure à celle du point de bulle de la membrane.

14. Conditionnement selon l'une quelconque des revendications 4 à 13, caractérisé en ce que la pression différentielle du point de bulle de la membrane (9,9a,9b) est d'envrion 4 bars, tandis que la récupération élastique de forme du récipient (1,1a,1b) après déformation est d'environ 0,003 bar.

## Claims

1. Method for distributing portions of sterile liquid enclosed in a container (1, 1a, 1b) under control of a microfiltering membrane (9, 9a, 9b) which is permeable to the liquid but impermeable to air in the wet state, interposed in the neck, without contamination of the liquid remaining between each distribution, said membrane (9, 9a, 9b) being moistened with the liquid from the inside of the container (1, 1a, 1b) prior to the first distribution and the desired quantity of liquid being distributed through the membrane (9, 9a, 9b) by exerting a pressure on the liquid within the container (1, 1a, 1b) characterized in that the liquid remaining in contact with the outer face of the membrane (9, 9a, 9b) is selectively sucked through this latter into the container (1, 1a, 1b) after distribution without suction of air and in that the membrane (9, 9a, 9b) is kept moistened with liquid between successive distributions.

2. Method according to claim 1, characterized in that the liquid is stored in a container (1, 1a, 1b) which is elastically deformable by hand and which produces between the faces of the membrane as a result of shape recovery after deformation a differential pressure lower in value than that of the bubble point of the membrane (9, 9a, 9b) but at least equal to the value which is necessary for suction of the liquid through the membrane (9, 9a, 9b).

3. Method according to either claim 1 or claim 2, characterized in that a volume of gas which is inert with respect to the liquid is enclosed in the container (1, 1a, 1b) with the liquid and compression of said volume of gas by manual deformation of the container (1, 1a, 1b) permits distribution of practically the entire quantity of liquid through the membrane (9, 9a, 9b).

4. Package for distributing sterile liquid in accordance with the method of one of claims 1 to 3, especially liquid which is sensitive to contact with air, including a container (1, 1a, 1b) having a wall elastically deformable by manual compression and a distributing aperture controlled by a microfiltering membrane (9, 9a, 9b) which is permeable to the liquid but impermeable to air in the wet state, said package characterized in that the force of elastic shape recovery of the wall of the container (1, 1a, 1b) after manual deformation produces between the faces of the membrane (9, 9a, 9b) a differential pressure lower than the differential pressure of the bubble point permitting the flow of air through the wet membrane, with the result that, after expulsion of liquid to the exterior through the membrane under the action of a pressure exerted externally on the container, the liquid remaining in contact with the outer face of the membrane when external pressure is stopped, returns selectively into the container through the membrane by suction under the action of depression produced within the container by the shape recovery of its wall without any possibility of concomitant suction of external air ; and in that it comprises means adapted to impose storage in the inverted position in order to keep the membrane moistened with liquid between successive distributions.

5. Package according to claim 4, characterized in that said container (1, 1a) comprises a rear surface (28) constituting an unstable bearing surface.

6. Package according to claim 4 or claim 5, characterized in that it comprises a rigid sheath (5, 5a) for protecting said container which has an elastically deformable wall (1, 1a), said sheath being cut away so as to permit access to said container in order to exert thereon a pressure adapted to expel a portion of the liquid.

7. Package according to claims 5 and 6, characterized in that said rigid sheath (5a) is open at its rear end, with the result that said container (1a) projects beyond the sheath in the rest position.

8. Package according to either claim 6 or claim 7, characterized in that said sheath (5b) is cut away in order not to afford a bearing surface for storage of the package.

9. Package according to claim 8, characterized in that said sheath (5) is widely bevelled from its rear end.

10. Package according to any one of claims 4 to 9, characterized in that the membrane (9, 9a) is covered externally by a distributing head (10, 10a) maintaining the outer face of the membrane against any deformation which would be prejudicial to its integrity while permitting discharge of the liquid which has passed through the membrane towards a capillary distribution nozzle, the total maximum volume of liquid between the outer face of the membrane and the tip of the nozzle being approximately 1 to 3 drops.

11. Package according to any one of claims 4 to 10, characterized in that it includes a cap (11, 11a) obturating the liquid distribution head (10, 10a), which constitutes a base (14, 14a) for storage of the container in an inverted position, thus maintaining the liquid of the container in permanent contact with the membrane.

12. Package according to either claim 4 or claim 10, characterized in that it comprises a tube (26) extending downwards to the bottom of the container and externally extended by a gooseneck terminated by a distributing head (10b) which contains the membrane (9b).

13. Package according to any one of claims 4 to 12, characterized int that it comprises a hand pup without any mechanical valve, incorporated in a shrinkable container which discharges the liquid of the container through a membrane which is permeable only to the liquid, forming selective non-return valve against the action of an elastic restoring force calibrated at a force lower than that of the bubble point of the membrane.

14. Package according to any one of claims 4 to 13, characterized in that the differential pressure of the bubble point of the membrane (9, 9a, 9b) is approximately 4 bar whereas the elastic shape recovery of the container (1, 1a, 1b) after deformation is approximately 0.003 bar.

## Patentansprüche

1. Verfahren zur portionsweisen Abgabe von steriler Flüssigkeit, die sich in einem Behälter (1, 1a, 1b) befindet, unter Kontrolle einer im Flaschenhals angeordneten Mikrofiltermembran (9, 9a, 9b), die für Flüssigkeiten permeabel, aber im feuchten Zustand für Luft impermeabel ist, so daß keine verunreinigung der nach jeder Abgabe übrigbleibenden Flüssigkeit stattfindet,
wobei die Membran (9, 9a, 9b) vor der ersten Abgabe mit der Flüssigkeit aus dem Inneren des Behälters (1, 1a, 1b) befeuchtet wird und die gewünschte Flüssigkeitsmenge durch die Membran (9, 9a, 9b) abgegeben wird, indem Druck auf die Flüssigkeit in dem Behälter (1, 1a, 1b) ausgeübt wird,
**dadurch gekennzeichnet,**
daß man nach der Abgabe selektiv die außen zurückbleibende und mit der Membran in Kontakt stehende Flüssigkeit durch die Membran (9, 9a, 9b) in den Behälter (1, 1a, 1b) einsaugt, ohne daß Luft eingesaugt wird,
und daß man die Membran (9, 9a, 9b) zwischen aufeinanderfolgenden Abgaben mit Flüssigkeit befeuchtet hält.

2. Verfahren gemäß Anspruch 1, dadurch gekennzeichnet, daß man die Flüssigkeit in einem per Hand elastisch deformierbaren Behälter (1, 1a, 1b) aufbewahrt, wobei die Wiederherstellung der Form nach der Deformation zwischen den Membranflächen einen Differenzdruck erzeugt, dessen Wert niedriger als der des Blasenpunktes der Membran (9, 9a, 9b), aber wenigstens so groß wie der zum Ansaugen von Flüssigkeit durch die Membran (9, 9a, 9b) nötige Wert ist.

3. Verfahren gemäß einem der Ansprüche 1 und 2, dadurch gekennzeichnet, daß man ein Volumen eines gegenüber der Flüssigkeit inerten Gases mit der Flüssigkeit in den Behälter (1, 1a, 1b) einbringt, dessen durch manuelle Deformation des Behälters (1, 1a, 1b) verursachte Kompression die Abgabe praktisch der gesamten Flüssigkeit durch die Membran (9, 9a, 9b) erlaubt.

4. Aufbewahrungs- und Abgabevorrichtung für sterile Flüssigkeiten nach dem Verfahren gemäß einem der Ansprüche 1 bis 3, insbesondere für Flüssigkeiten, die gegen Kontakt mit Luft empfindlich sind,
welche einen Behälter (1, 1a, 1 b) mit einer durch manuelle Kompression elastisch deformierbaren Wand aufweist,
deren Abgabeöffnung durch eine Mikrofiltermembran (9, 9a, 9b), die für Flüssigkeiten permeabel, aber im feuchten Zustand für Luft impermeabel ist, kontrolliert wird;
dadurch gekennzeichnet, daß die Kraft der elastischen Wiederherstellung der Form der Wand des Behälters (1, 1a, 1b) nach der manuellen Deformation zwischen den Flächen der Membran (9, 9a, 9b) einen niedrigeren Differenzdruck erzeugt als der Differenzdruck des Blasenpunktes, welcher der Luft ermöglicht, die feuchte Membran zu durchqueren, so daß, nach dem Ausstoß von Flüssigkeit durch die Membran nach außen, unter dem Einfluß eines von außen auf den Behälter ausgeübten Druckes, die mit der Außenseite der Membran in Kontakt stehende Flüssigkeit selektiv durch die Membran in den Behälter zurückfließt sobald der äußere Druck aufhört, wobei dieses Einsaugen durch den Unterdruck verursacht wird, der in dem Behälter durch die Wiederherstellung der Form seiner Wand erzeugt wird, ohne daß Luft von außen miteingesaugt werden kann;
und daß sie geeignete Mittel aufweist, um eine Lagerung in einer auf dem Kopf stehenden Position zu erzwingen, so daß die Membran zwischen aufeinanderfolgenden Abgaben mit Flüssigkeit befeuchtet bleibt.

5. Vorrichtung gemäß Anspruch 4, dadurch gekennzeichnet, daß der Behälter (1, 1a) eine hintere Fläche (28) aufweist, die eine labile Auflagefläche bildet.

6. Vorrichtung gemäß Anspruch 4 oder 5, dadurch gekennzeichnet, daß sie eine starre Hülle (5, 5a) zum Schutz des aus einer elastisch deformierbaren Wand bestehenden Behälters (1, 1a) aufweist, wobei die Hülle so ausgeschnitten ist, daß der Zugang zum Behälter ermöglicht wird, um auf ihn einen geeigneten Druck zum Ausstoß einer Flüssigkeitsmenge auszuüben.

7. Vorrichtung gemäß den Ansprüchen 5 und 6, dadurch gekennzeichnet, daß die starre Hülle (5a) an ihrem hinteren Ende offen ist, so daß der Behälter (1a) im Ruhezustand über die Hülle hinausragt.

8. Vorrichtung gemäß einem der Ansprüche 6 und 7, dadurch gekennzeichnet, daß die Hülle (5b) so geschnitten ist, daß sie keine Standfläche für die Lagerung der Vorrichtung bietet.

9. Vorrichtung gemäß Anspruch 8, dadurch gekennzeichnet, daß die Hülle (5) von ihrem hinteren Ende her weitgehend als Schrägfläche zugeschnitten ist.

10. Vorrichtung gemäß einem der Ansprüche 4 bis 9, dadurch gekennzeichnet, daß die Membran (9, 9a) außen von einem Abgabekopf (10, 10a) bedeckt ist, der die Außenseite der Membran vor jeder für ihre Unversehrtheit nachteiligen Verformung bewahrt, während gleichzeitig der Abfluß der Flüssigkeit, die die Membran durchquert hat, in ein kapillares Abgaberöhrchen ermöglicht wird, wobei das gesamte maximale Flüssigkeitsvolumen zwischen der Außenseite der Membran und dem Ende des Röhrchens etwa 1 bis 3 Tropfen beträgt.

11. Vorrichtung gemäß einem der Ansprüche 4 bis 10, dadurch gekennzeichnet, daß sie eine den Flüssigkeitsabgabekopf (10, 10a) verschließende Haube (11, 11a) aufweist, die einen Sockel (14, 14a) zur Lagerung des Behälters in einer auf dem Kopf stehenden Position bildet und die Flüssigkeit im Behälter so in ständigem Kontakt mit der Membran bleibt.

12. Vorrichtung gemäß einem der Ansprüche 4 oder 10, dadurch gekennzeichnet, daß sie ein Rohr (26) aufweist, das bis zum Boden des Behälters reicht und das außen durch einen Schwanenhals verlängert ist, der in einem Abgabekopf (10b) endet, welcher die Membran (9b) enthält.

13. Vorrichtung gemäß einem der Ansprüche 4 bis 12, dadurch gekennzeichnet, daß sie eine in einem zusammenpreßbaren Behälter angeordnete manuelle Pumpe ohne mechanisches Ventil aufweist, die die Flüssigkeit vom Behälter durch eine nur für Flüssigkeit permeable Membran fördert, wobei ein selektives Rückschlagventil gegen die Wirkung einer elastischen Rückstellkraft gebildet wird, die auf eine geringere Kraft eingestellt ist als die des Blasenpunktes der Membran.

14. Vorrichtung gemäß einem der Ansprüche 4 bis 13, dadurch gekennzeichnet, daß der Differenzdruck des Blasenpunktes der Membran (9, 9a, 9b) etwa 4 bar beträgt, während die elastische Wiederherstellung der Form des Behälters (1, 1a, 1b) nach der Deformation etwa 0,003 bar entspricht.
